# FASCICULE DE BREVET EUROPEEN

(11) **EP 1 162 982 B1**
(45) Date de publication et mention de la délivrance du brevet: **09.02.2005**
(21) Numéro de dépôt: 00910993.5
(22) Date de dépôt: 17.03.2000
(51) Int. Cl.: A61K 31/7088, C07H 21/00, A61P 35/00

(54) **UTILISATION D'OLIGONUCLEOTIDES STABILISES POUR LA PREPARATION D'UN MEDICAMENT A ACTION ANTITUMORALE**
VERWENDUNG VON STABILISIERTEn OLIGONUCLEOTIDEn ZUR HERSTELLUNG VON ANTITUMORALen ARZNEIMITTELn
USE OF STABILISED OLIGONUCLEOTIDES FOR PREPARING A MEDICament WITH ANTITUMOUR ACTIVITY

(30) Priorité: 19.03.1999 FR 9903433
(43) Date de publication de la demande: 19.12.2001
(73) Titulaire: ASSISTANCE PUBLIQUE, HOPITAUX DE PARIS, 75004 Paris (FR); INSTITUT NATIONAL DE LA SANTE ET DE LA RECHERCHE MEDICALE (INSERM), 75654 Paris Cédex 13 (FR)
(72) Inventeur: CARPENTIER, Antoine, F-75007 Paris (FR)
(74) Mandataire: Vialle-Presles, Marie José
(86) Numéro de dépôt international: PCT/FR2000/000676
(87) Numéro de publication internationale: WO 2000/056342

(56) Documents cités:
- EP-A- 0 468 520
- EP-A- 0 855 184
- WO-A-94/25588
- WO-A-95/02051
- WO-A-95/08350
- WO-A-95/32987
- WO-A-96/02555
- WO-A-97/44346
- WO-A-98/18810
- WO-A-98/55495
- WO-A-99/12027
- WO-A-99/26634
- WO-A-99/51259
- US-A- 5 734 033
- US-A- 5 874 416
- CONNELL, Y. S. ET AL: "Anti-tumor activity of a CpG-containing oligodeoxynucleotide (ODN) in athymic mice." PROCEEDINGS OF THE AMERICAN ASSOCIATION FOR CANCER RESEARCH ANNUAL MEETING, (MARCH, 1999) VOL. 40, PP. 299. MEETING INFO.: 90TH ANNUAL MEETING OF THE AMERICAN ASSOCIATION FOR CANCER RESEARCH PHILADELPHIA, PENNSYLVANIA, USA APRIL 10-14, 1999 AMERICAN, XP000857678
- SONEHARA K ET AL: "Hexamer palindromic oligonucleotides with 5'-CG-3' motif(s) induce production of interferon." JOURNAL OF INTERFERON AND CYTOKINE RESEARCH, (1996 OCT) 16 (10) 799-803., XP000863569

### LISTE DE SEQUENCES

<110> ASSISTANCE PUBLIQUE- HOPITAUX DE PARIS
   UNIVERSITE PIERRE ET MARIE CURIE- PARIS VI
   INSTITUT NATIONAL DE LA SANTE ET DE LA RECHERCHE M CARPENTIER, Antoine
<120> UTILISATION D'OLIGONUCLEOTIDES STABILISES POUR LA PREPARATION D'UN MEDICAMENT A ACTION ANTITUMORALE
<130> 10208EXT
<140>
   <141>
<160> 48
<170> PatentIn Ver. 2.1
<210> 1
   <211> 22
   <212> ADN
   <213> Séquence artificielle
<220>
   <223> Description de la séquence artificielle: oligodésoxynucléotide
<400> 1
   tgactgtgaa ggttagagat ga                                          22
<210> 2
   <211> 22
   <212> ADN
   <213> Séquence artificielle
<220>
   <223> Description de la séquence artificielle: oligodésoxynucléotide
<400> 2
   tgactgtgaa cgttcgagat ga                                          22
<210> 3
   <211> 22
   <212> ADN
   <213> Séquence artificielle
<220>
   <223> Description de la séquence artificielle: oligodésoxynucléotide
<400> 3
   tgactgtgaa cgttccagat ga 22
<210> 4
   <211> 20
   <212> ADN
   <213> Séquence artificielle
<220>
   <223> Description de la séquence artificielle: oligodésoxynucléotide
<400> 4
   tgccagtaac gttacgtgac 20
<210> 5
   <211> 20
   <212> ADN
   <213> Séquence artificielle
<220>
   <223> Description de la séquence artificielle: oligodésoxynucléotide
<400> 5
   tgccagtaac gttaggtgac 20
<210> 6
   <211> 20
   <212> ADN
   <213> Séquence artificielle
<220>
   <223> Description de la séquence artificielle: oligodésoxynucléotide
<400> 6
   tgccagtaac gttgtgtgac 20
<210> 7
   <211> 20
   <212> ADN
   <213> Séquence artificielle
<220>
   <223> Description de la séquence artificielle: oligodésoxynucléotide
<400> 7
   tgccagtaac gttccgtgac 20
<210> 8
   <211> 20
   <212> ADN
   <213> Séquence artificielle
<220>
   <223> Description de la séquence artificielle: oligodésoxynucléotide
<400> 8
   tgccagtgac gtcatgtgac 20
<210> 9
   <211> 22
   <212> ADN
   <213> Séquence artificielle
<220>
   <223> Description de la séquence artificielle: oligodésoxynucléocide
<400> 9
   tgactgtgaa cgttatagat ga 22
<210> 10
   <211> 20
   <212> ADN
   <213> Séquence artificielle
<220>
   <223> Description de la séquence artificielle: oligodésoxynucléotide
<400> 10
   tgccagtaac gttatgtgac 20
<210> 11
   <211> 20
   <212> ADN
   <213> Séquence artificielle
<220>
   <223> Description de la séquence artificielle: oligodésoxynucléotide
<400> 11
   tgccagtaac gttaagtgac 20
<210> 12
   <211> 20
   <212> ADN
   <213> Séquence artificielle
<220>
   <223> Description de la séquence artificielle: oligodésoxynucléotide
<400> 12
   tgccagtaac gttctgtgac 20
<210> 13
   <211> 20
   <212> ADN
   <213> Séquence artificielle
<220>
   <223> Description de la séquence artificielle: oligodésoxynucléotide
<400> 13
   tgccagtaac gttttgtgac 20
<210> 14
   <211> 20
   <212> ADN
   <213> Séquence artificielle
<220>
   <223> Description de la séquence artificielle: oligodésoxynucléotide
<400> 14
   gtatgacgac gtcatctagc 20
<210> 15
   <211> 20
   <212> ADN
   <213> Séquence artificielle
<220>
   <223> Description de la séquence artificielle: oligodésoxynucléotide
<400> 15
   tactgcagac gtcattatgc 20
<210> 16
   <211> 20
   <212> ADN
   <213> Séquence artificielle
<220>
   <223> Description de la séquence artificielle: oligodésoxynucléotide
<400> 16
   ataacgttat gtaacgttat 20
<210> 17
   <211> 20
   <212> ADN
   <213> Séquence artificielle
<220>
   <223> Description de la séquence artificielle: oligodésoxynucléotide
<400> 17
   atgacgtcat gtgacgtcat 20
<210> 18
   <211> 20
   <212> ADN
   <213> Séquence artificielle
<220>
   <223> Description de la séquence artificielle: oligodésoxynucléotide
<400> 18
   atgacgtcat gtaacgttat 20
<210> 19
   <211> 20
   <212> ADN
   <213> Séquence artificielle
<220>
   <223> Description de la séquence artificielle : oligodésoxynucléotide
<400> 19
   tgaacgttat tgaacgttat 20
<210> 20
   <211> 20
   <212> ADN
   <213> Séquence artificielle
<220>
   <223> Description de la séquence artificielle : oligodésoxynucléotide
<400> 20
   tggacgtcat tggacgtcat 20
<210> 21
   <211> 20
   <212> ADN
   <213> Séquence artificielle
<220>
   <223> Description de la séquence artificielle: oligodésoxynucléotide
<400> 21
   tggacgtcat tgaacgttat 20
<210> 22
   <211> 20
   <212> ADN
   <213> Séquence artificielle
<220>
   <223> Description de la séquence artificielle: oligodésoxynucléotide
<400> 22
   aggacgtcaa tgaacgttaa 20
<210> 23
   <211> 20
   <212> ADN
   <213> Séquence artificielle
<220>
   <223> Description de la séquence artificielle: oligodésoxynucléotide
<400> 23
   agaacgttaa tgaacgttaa 20
<210> 24
   <211> 20
   <212> ADN
   <213> Séquence artificielle
<220>
   <223> Description de la séquence artificielle: oligodésoxynucléotide
<400> 24
   aggacgtcaa tggacgtcaa 20
<210> 25
   <211> 20
   <212> ADN
   <213> Séquence artificielle
<220>
   <223> Description de la séquence artificielle: oligodésoxynucléotide
<400> 25
   aggacgtctt gtaacgtttt 20
<210> 26
   <211> 20
   <212> ADN
   <213> Séquence artificielle
<220>
   <223> Description de la séquence artificielle: oligodésoxynucléotide
<400> 26
   agaacgtttt gtaacgtttt 20
<210> 27
   <211> 19
   <212> ADN
   <213> Séquence artificielle
<220>
   <223> Description de la séquence artificielle: oligodésoxynucléotide
<400> 27
   aggacgtctt gtgacgttt 19
<210> 28
   <211> 20
   <212> ADN
   <213> Séquence artificielle
<220>
   <223> Description de la séquence artificielle: oligodésoxynucléotide
<400> 28
   ttgacgtcct ttaacgttct 20
<210> 29
   <211> 20
   <212> ADN
   <213> Séquence artificielle
<220>
   <223> Description de la séquence artificielle: oligodésoxynucléotide
<400> 29
   ttaacgttct ttaacgttct 20
<210> 30
   <211> 20
   <212> ADN
   <213> Séquence artificielle
<220>
   <223> Description de la séquence artificielle: oligodésoxynucléotide
<400> 30
   ttgacgtcct ttgacgtcct 20
<210> 31
   <211> 26
   <212> ADN
   <213> Séquence artificielle
<220>
   <223> Description de la séquence artificielle: oligodésoxynucléotide
<400> 31
   taaacgttat aacgttataa cgttat 26
<210> 32
   <211> 26
   <212> ADN
   <213> Séquence artificielle
<220>
   <223> Description de la séquence artificielle: oligodésoxynucléotide
<400> 32
   tagacgtcat gacgtcatga cgtcat 26
<210> 33
   <211> 26
   <212> ADN
   <213> Séquence artificielle
<220>
   <223> Description de la séquence artificielle: oligodésoxynucléotide
<400> 33
   taaacgttat aacgttatga cgtcat 26
<210> 34
   <211> 26
   <212> ADN
   <213> Séquence artificielle
<220>
   <223> Description de la séquence artificielle: oligodésoxynucléotide
<400> 34
   taaacgttat gacgtcatga cgtcat 26
<210> 35
   <211> 26
   <212> ADN
   <213> Séquence artificielle
<220>
   <223> Description de la séquence artificielle: oligodésoxynucléotide
<400> 35
   tagacgtcat aacgttataa cgttat 26
<210> 36
   <211> 26
   <212> ADN
   <213> Séquence artificielle
<220>
   <223> Description de la séquence artificielle: oligodésoxynucléotide
<400> 36
   taaacgttaa aacgttaaaa cgttaa 26
<210> 37
   <211> 26
   <212> ADN
   <213> Séquence artificielle
<220>
   <223> Description de la séquence artificielle: oligodésoxynucléotide
<400> 37
   tagacgtcaa gacgtcaaga cgtcaa 26
<210> 38
   <211> 26
   <212> ADN
   <213> Séquence artificielle
<220>
   <223> Description de la séquence artificielle: oligodésoxynucléotide
<400> 38
   taaacgttaa aacgttaaga cgtcaa 26
<210> 39
   <211> 26
   <212> ADN
   <213> Séquence artificielle
<220>
   <223> Description de la séquence artificielle: oligodésoxynucléotide
<400> 39
   taaacgttaa gacgtcaaga cgtcaa 26
<210> 40
   <211> 26
   <212> ADN
   <213> Séquence artificielle
<220>
   <223> Description de la séquence artificielle: oligodésoxynucléotide
<400> 40
   tagacgtcaa aacgttaaaa cgttaa 26
<210> 41
   <211> 26
   <212> ADN
   <213> Séquence artificielle
<220>
   <223> Description de la séquence artificielle: oligodésoxynucléotide
<400> 41
   taaacgttct aacgttctaa cgttct 26
<210> 42
   <211> 26
   <212> ADN
   <213> Séquence artificielle
<220>
   <223> Description de la séquence artificielle: oligodésoxynucléotide
<400> 42
   tagacgtcct gacgtcctga cgtcct 26
<210> 43
   <211> 26
   <212> ADN
   <213> Séquence artificielle
<220>
   <223> Description de la séquence artificielle: oligodésoxynucléotide
<400> 43
   taaacgttct aacgttctga cgtcct 26
<210> 44
   <211> 28
   <212> ADN
   <213> Séquence artificielle
<220>
   <223> Description de la séquence artificielle: oligodésoxynucléotide
<400> 44
   taaacgtttt aaacgtttta aacgtttt 28
<210> 45
   <211> 28
   <212> ADN
   <213> Séquence artificielle
<220>
   <223> Description de la séquence artificielle: oligodésoxynucléotide
<400> 45
   tagacgtctt agacgtctta gacgtctt 28
<210> 46
   <211> 28
   <212> ADN
   <213> Séquence artificielle
<220>
   <223> Description de la séquence artificielle: oligodésoxynucléotide
<400> 46
   taaacgtttt aaacgtttta gacgtctt 28
<210> 47
   <211> 26
   <212> ADN
   <213> Séquence artificielle
<220>
   <223> Description de la séquence artificielle: oligodésoxynucléotide
<400> 47
   tcaacgttat aacgttataa cgttat 26
<210> 48
   <211> 26
   <212> ADN
   <213> Séquence artificielle
<220>
   <223> Description de la séquence artificielle: oligodésoxynucléotide
<400> 48
   tcgacgtcat gacgtcatga cgtcat                                      26

## Description

La présente invention se rapporte à l'utilisation d'oligonucléotides stabilisés pour la préparation d'un médicament à action antitumorale.

Le traitement efficace des cancers reste l'un des défis majeurs de la médecine d'aujourd'hui.

L'efficacité des thérapeutiques conventionnelles chirurgicales ou à visée cytolytique (chimiothérapie et radiothérapie) reste très limitée dans de nombreux cancers.

Pour les astrocytomes par exemple, dont le traitement repose principalement sur l'exérèse chirurgicale et l'irradiation cérébrale locale, la médiane de survie est seulement de 4 à 6 mois après exérèse chirurgicale et de 8 à 10 mois avec l'association chirurgie et radiothérapie. Une chimiothérapie complémentaire allonge la survie chez les patients de moins de 60 ans, mais de façon très modeste, de l'ordre de 3 mois. Sous ce triple traitement, la médiane de survie reste inférieure à deux ans pour le grade histologique III (astrocytome anaplasique) et inférieure à 1 an pour le grade IV (glioblastome). La mortalité pour ces deux groupes est de 100% (Daumas-Duport C. *et al.* (1988), Cancer **62**(10) pp 2152-65).

La stimulation du système immunitaire dans le traitement des cancers est une idée ancienne et de très nombreux produits ont été testés, comme par exemple les extraits bactériens (Jaeckle K. A. *et al.* (1990), *J. Clin. Oncol.* **8**(8) pp 1408-18), l'ADN bactérien, notamment celui de *Mycobacterium bovis* (MY-1) (Tokunaga T. *et al.* (1984), *JNCI* **72** pp 955-62). MY-1 est cependant inefficace pour augmenter la survie dans un modèle de gliome chez la souris (Nakaichi M. *et al.* (1995), *J. Vet. Med. Sci.* **57**(3) pp 583-5). L'IL2 (Herrlinger U. *et al*. (1996), *J. Neurooncol.* **27**(3) pp 193-203) et plus récemment l'IL12 (Kishima H. *et al.* (1998), *Br. J. Cancer* **78**(4) pp 446-53 ; Jean W. C. *et al.* (1998), *Neurosurgery* **42**(4) pp 850-6) ont également été étudiés.

Malheureusement, la plupart de ces produits ont une efficacité limitée ou une toxicité inacceptable et à ce jour, seul le BCG de *Mycobacterium bovis* a abouti à des applications cliniques, mais seulement dans des indications très limitées de cancers de la vessie (Soloway M. S. *et al.* (1988), *Urol. Clin. North Am.* **15** pp 661-9).

Les oligonucléotides sont des polymères, formés par la combinaison de bases puriques ou pyrimidiques et de sucres, notamment des ribonucléotides ou des désoxy-ribonucléotides. A l'état naturel, les liaisons sont des liaisons phosphoesters sensibles aux nucléases de l'organisme humain. Ainsi les oligonucléotides présentent une demi-vie très courte (de l'ordre de la minute) quand ils sont injectés chez l'homme, ce qui limite leurs effets biologiques. Aussi, plusieurs études ont cherché à stabiliser les oligonucléotides en modifiant leur structure chimique pour les rendre résistants aux nucléases. Plusieurs types d'oligonucléotides stabilisés ont ainsi été créés comme, entre autres, les phosphorothioates ou les méthylphosphonates, (Crooke R. M. (1991), *Anti-Cancer Drug Design* **6** pp 609-46). Les plus fréquemment utilisés sont les oligonucléotides phosphorothioates.

Certains oligodésoxynucléotides et notamment certains oligodésoxynucléotides synthétiques possèdent parfois des effets biologiques propres, en dehors de leurs propriétés antisens classiques.

Ainsi certains oligodésoxynucléotides, indépendamment de toute séquence antisens connue, stimulent, *in vi tro* et *in vivo,* la prolifération des lymphocytes B, l'activité des cellules NK et induisent la sécrétion par ces cellules d'IFN α, d'IFN β, d'IFN γ, d'IL6, d' IL12 ou de TNF α (Yamamoto *S. et al.* (1992), *J*. *Immunol.* **148**(12) pp 4072-6 ; Yamamoto T. *et al.* (1994), *Microbiol. Immunol.* **38**(10), pp 831-6 ; Yi A. K. *et al.* (1996),. *J. Immunol.* **157**(12) pp 5394-402 ; Ballas Z. K. *et al.* (1996), *J. Immunol.* **157**(5) pp 1840-5 ; Cowdery J. S. *et al.* (1996), *J. Immunol.* **156**(12) pp 4570-5 ; Stacey K. J. *et al.* (1996), *J. Immunol.* **157**(5) pp 2116-22). L'ensemble de ces cytokines oriente vers une réponse immunitaire de type Th1 (Chu R.S. *et al.* (1997), *J. Exp. Med.* **186**(10) pp 1623-31).

Des Auteurs ont montré que les propriétés immunostimulantes de ces oligodésoxynucléotides sont en grande partie dépendantes de motifs CG non méthylés (dinucléotides CpG non méthylés) qui sont sous-représentés dans l'ADN de mammifères (Kuramoto E. *et al.* (1992), *Jpn. J. Cancer Res.,* **83** pp 1128-31).

Si les Auteurs s'accordent sur le fait que la séquence CG non méthylée est indispensable et que les deux nucléotides adjacents au motif CG, sont également cruciaux pour l'activité immunostimulante, les données publiées sur la nature des séquences adjacentes sont contradictoires.

En effet, Krieg A. M. *et al.* ((1996), *Antisense Nucleic Acid Drug Dev.* **6**(2) pp 133-9 revendiquent un motif hexamérique du type 5'purine purine CG pyrimidine pyrimidine 3', alors que la demande EP 468 520 revendique un motif hexamérique palindromique. La Demande internationale WO 9855495 montre, que tous les hexamères tels que définis par Krieg *et al.* 1996 (précité) ne sont pas immunostimulants et qu'il convient plutôt de définir des octamères, de séquence 5' purine purine CG pyrimidine pyrimidine CC-3' ou de séquence 5'-purine purine CG pyrimidine pyrimidine CG-3', pour avoir une activité immunostimulante.

D'autres oligodésoxynucléotides immunostimulants, qui ne sont pas définis comme des oligonucléotides possédant un motif CG non méthylé ont été décrits dans la Demande EP 855 184 : ils comprennent la séquence de fixation de facteurs de transcription eucaryote tels que NFκB ou la famille AP-1. Parmi ces oligonucléotides, certains comprennent néanmoins le motif CG non méthylé.

L'utilisation des propriétés immunostimulantes des oligodésoxynucléotides comprenant un motif de type CG non méthylé, fait l'objet de recherches dans des domaines très variés :
(1) dans le domaine de la vaccination ils sont utilisés, en association avec l'antigène, comme adjuvant pour stimuler spécifiquement une réponse immunitaire de type Th1 (Davis H. L. *et al.* (1998), *The Journal of Immunology* **160**(2) pp 870-6 ; demande EP 855 184 ; Demande internationale WO 9818810 au nom de The University of Iowa Research Foundation dont A. M. Krieg est l'un des inventeurs; Demande internationale WO 9855495),
(2) dans le domaine de l'allergie, ils sont utilisés seuls pour moduler la réponse immunitaire (Demandes internationales WO 9818810 et WO 9855495) et
(3) dans le domaine du cancer, ils sont utilisés :
   - soit en association avec un antigène tumoral, comme adjuvant d'un vaccin antitumoral (demande EP 855 184; Weiner G. J. *et al.* (1996), *Proc. Natl. Acad. Sci.* **94,** pp 10833-7 ; Wooldridge J. E. *et al.* (1997) *Blood* **89**(8) pp 2994-8),
   - soit seuls comme antitumoraux (Connell *et al.* (1999), *Proceedings of the American association for Cancer Research* **40** pp 299 ; demande EP 468 520; Carpentier A. F. *et al.* (1999), *Cancer Research* **59**, pp 5429-5432.

Dans ce dernier cas, l'activité antitumorale de quelques séquences seulement, parmi celles décrites, a été effectivement démontrée :
- Weiner G. J. *et al*. et Wooldridge J.E. *et al.* (déjà cités) qui utilisent un oligonucléotide comprenant un motif CG non méthylé de séquence 5'-TCTCCC AGCGTGCGCCAT-3' montrent que cet oligonucléotide ne possède aucun effet antitumoral lorsqu'il est utilisé seul,
- Carpentier *et al.*, Tokunaga *et al.* et Connell *et al.* (précités) qui utilisent respectivement un oligonucléotide phosphorothioate du type octamérique (5'TGACTGTGAACGTTCGAGATGA3'), un oligonucléotide hexamérique palindromique non stabilisé (5' ACCGAT GACGTCGCCGGTGACGGCACCACGACGACGGCCACGTGCT 3') et un oligonucléotide phosphorothioate hexamérique du type 5' purine purine CG pyrimidine pyrimidine 3', montrent que lesdits oligonucléotides possèdent une activité antitumorale.

Il ressort de l'Art antérieur qu'en dehors du motif CG non méthylé, la nature exacte des séquences actives de ces oligodésoxynucléotides immunostimulants, pour l'obtention d'une activité antitumorale, n'est pas clairement définie ; en particulier, les données publiées sur la nature des séquences adjacentes au motif CG non méthylé (2 bases en 5' et 2 bases en 3' (motifs hexamériques) ou 4 bases en 3' (motif octamérique)) sont contradictoires.

Des études récentes rapportées par Hartmann G. *et al.* ((2000), *The Journal of Immunology* 164 pp 1617-24) donnent des explications sur la difficulté de définir la séquence de tels oligonucléotides.

En effet, les Auteurs montrent que, selon la nature de leur séquence, les oligodésoxynucléotides immunostimulants ont des effets différentiels sur l'activation NK, la prolifération des lymphocytes B, la sécrétion d'IL12, d'IL6 et d'INFγ.
Ces données indiquent que tous les oligodésoxynucléotides immunostimulants ne sont pas équivalents et efficaces pour toutes les utilisations telles que définies ci-dessus puisqu'il faut stimuler des compartiments du système immunitaire différents afin d'obtenir l'activité désirée : adjuvante, antiallergique ou antitumorale.

De plus, les mécanismes immunitaires de rejet tumoral sont mal connus et les données de stimulation des compartiments du système immunitaire, *in vitro*, telles que définies ci-dessus ne permettent pas de prévoir à l'avance l'efficacité antitumorale d'un oligonucléotide donné et il importe donc de tester leur activité antitumorale *in vivo*.

En outre, la toxicité d'oligonudésoxy-nucléotides contenant un motif de type CG a été rapportée lors de leur utilisation par voie systémique (IV et IP) et est aussi à prendre en compte pour des applications thérapeutiques(Demande EP 855 184).

En conséquence, les oligonucléotides immunostimulants comprenant un motif CG de l'Art antérieur (motif hexamérique palindromique, motif 5' purine purine CG pyrimidine pyrimidine 3' ou motif octamérique) qui présentent des activités antitumorales variables et aléatoires et sont toxiques, ne permettent pas de définir un ensemble de séquences immunostimulantes non toxiques, efficaces, pour une utilisation antitumorale.
Or, les Inventeurs ont montré, de manière surprenante, que certaines paires de bases en 3' du motif 5' purine purine CG pyrimidine pyrimidine 3' participent, de manière cruciale, à une activité antitumorale optimale.

En conséquence, les Inventeurs se sont donnés pour but de pourvoir à un ensemble de séquences d'oligonucléotides immunostimulants qui répondent mieux aux besoins de la pratique en ce qu'ils :
- possèdent une activité antitumorale optimale,
- ne sont pas toxiques et
- sont adaptés à une utilisation antitumorale chez l'homme ou l'animal.

Ainsi la présente invention a pour objet, l'utilisation d'oligonucléotides stabilisés qui comprennent au moins un motif octamérique du type : AACGTTAT, pour la préparation d'un médicament à action antitumorale.

Au sens de la présente invention, on entend par oligonucléotide, un oligodésoxynucléotide.

Dans un autre mode de réalisation préféré de l'invention, l'une au moins des bases du motif octamérique décrit ci-dessus peut être modifiée, notamment l'une au moins des cytosines peut être remplacée par une 5-bromocytosine.

Dans un autre mode de réalisation préféré de l'invention, l'oligonucléotide stabilisé est sélectionné dans le groupe constitué par les séquences SEQ ID NO :9, 10, 16, 18, 19, 21, 31, 33, 34, 35 et 47.

Conformément à l'invention, les oligonucléotides stabilisés sont sélectionnés notamment dans le groupe constitué par les oligonucléotides phosphorothioates, les oligonucléotides phosphorodithioates, les oligonucléotides mixtes phosphodiester-phosphorothioates, les oligonucléotides méthylphosphonates ou les oligonucléotides dont au moins une extrémité a été stabilisée (Crooke R. M. (1991), *AntiCancer Drug Design,* **6** pp 609-46). De préférence, les oligonucléotides stabilisés utilisés selon la présente invention sont des phosphorothioates.

Conformément à l'invention, les oligonucléotides stabilisés peuvent être utilisés sous forme de simple brin ou de double brin.

De préférence, les oligonucléotides stabilisés peuvent avoir n'importe quelle longueur supérieure à 8 bases ou 8 paires de bases, de préférence plus de 20 bases ou de 20 paires de bases. De manière préférée lesdits oligonucléotides comprennent entre 20 et 100 nucléotides.

Conformément à la présente invention, les oligonucléotides peuvent comprendre plusieurs motifs octamériques tels que définis ci-dessus adjacents ou non ; ils peuvent comprendre également d'autres séquences biologiquement actives, comme des séquences antisens. Les séquences octamériques peuvent elles-mêmes être incluses dans des séquences antisens.

La présente invention a également pour objet l'utilisation des oligonucléotides stabilisés tels que définis ci-dessus, pour la préparation de médicaments destinés au traitement des cancers chez l'homme, quels que soient leur nature et leur degré d'anaplasie, en particulier les cancers des systèmes nerveux central et périphérique, notamment les astrocytomes, les glioblastomes, les médulloblastomes, les neuroblastomes, les mélanomes et les carcinomes.

Les oligonucléotides stabilisés, peuvent avantageusement être couplés par des liaisons covalentes, ioniques ou faibles à une molécule susceptible d'augmenter l'affinité tumorale, comme par exemple un anticorps spécifique du tissu tumoral.

Les oligonucléotides stabilisés sont utilisés préférentiellement par voie intra-tumorale, mais ils peuvent être administrés également par toutes autres voies, éventuellement par voies multiples, notamment par voies intraveineuse, intrapéritonéale, topique, transdermique, sous-cutanée, intra-artérielle, pulmonaire, naso-pharyngée ou orale, en solution, en suspension aqueuse ou huileuse, en poudre ou sous toute forme pharmaceutiquement acceptable.

Ils peuvent être administrés en une ou plusieurs fois ou en libération continue, notamment au moyen de micropompes osmotiques ou associés à tout moyen physique ou chimique, notamment à des agents encapsulants comme les systèmes de dispersion colloïdaux et les polymères, afin d'avoir une dose thérapeutiquement efficace au site tumoral.

Les doses efficaces seront déterminées en fonction de l'âge, de l'état de santé, du poids du patient et du type de cancer à traiter. Typiquement, les doses efficaces chez l'homme sont telles que, dans le cas d'une injection intra-tumorale, l'on obtienne une dose d'oligonucléotide de 10 à 1000 µg/g de tumeur, au moins dans une partie de la tumeur.

Conformément à l'invention, l'utilisation des oligonucléotides peut être combinée avec d'autres thérapies, notamment la chirurgie, la radiothérapie, la chimiothérapie, l'immunothérapie et des thérapies différenciantes.

Également conformément à l'invention, lesdits oligonucléotides sont associés avec des cellules du système immunitaire, telles que des macrophages, des lymphocytes ou des cellules présentatrices d'antigène, des adjuvants de l'immunité, des cytokines, des anticorps anti-tumoraux, des extraits tumoraux, des antigènes tumoraux ou des cellules tumorales normales, irradiées ou génétiquement modifiées.

Outre les dispositions qui précèdent, l'invention comprend encore d'autres dispositions, qui ressortiront de la description qui va suivre, qui se réfère aux exemples de réalisation de l'utilisation, objet de la présente invention, ainsi qu'aux dessins annexés, dans lesquels:
- la figure 7 illustre l'effet de la stabilisation d'un oligonucléotide (SEQ ID NO:9 5'-TGACTGTGAACGTTATAGATGA-3') par une liaison du type phosphorothioate (PT), phosphodiester (PDE), méthylphosphonate (MP) ; phosphodiester stabilisé en 3' par une base didésoxycytosine (3') ou mixte : phosphodiester avec les 3 premières liaisons en 5' et les trois dernières liaisons en 3' de type phosphorothioates (mixte), sur l'activité antitumorale dans un modèle de tumeurs gliales sous-cutanées. A J2 après l'injection des cellules tumorales, les groupes d'animaux reçoivent par voie sous cutanée, au niveau du site tumoral, du chlorure de sodium (témoin Nacl, n= 9) ou 50 µg des oligonucléotides PT (n= 9) , PDE (n= 8), MP (n= 9), 3' (n= 7) et mixte (n= 9). Le volume de la tumeur est évalué à J10. Les résultats sont exprimés en moyenne ± s.e.m..
- les figures 8 à 11 illustrent l'effet des séquences 5'-purine-purine-CG-pyrimidine-pyrimidine-X₁X₂-3' sur la modulation de l'activité antitumorale dans un modèle de tumeurs gliales sous-cutanées. A J2 après l'injection des cellules tumorales, les groupes d'animaux (n= 6) reçoivent par voie sous cutanée, au niveau du site tumoral, du chlorure de sodium (témoin Nacl) ou 50 µg des oligonucléotides (SEQ ID NO :2 à 13). Le volume de la tumeur est évalué à J8 (figure 8 à 10) ou à J10 (figure 11). Les résultats sont exprimés en moyenne ± s.e.m. :
   - la figure 8 illustre l'effet des séquences des oligonucléotides sur l'efficacité antitumorale,
   - la figure 9 illustre l'effet de la séquence du motif hexamérique 5'-purine-purine-CG-pyrimidine-pyrimidine-3' et des séquences adjacentes sur l'efficacité antitumorale des oligonucléotides,
   - la figure 10 illustre l'effet des 2 bases (X₁X₂) adjacentes à la séquence 3' du motif hexamérique 5'-purine-purine-CG-pyrimidine-pyrimidine-3' sur l'efficacité antitumorale des oligonucléotides.

Les exemples qui suivent illustrent l'invention, sans toutefois la limiter à ces modes de réalisation particuliers.

### Exemple 12 : Effet de la stabilisation d'un oligonucléotide (SEQ ID N :9 5'-TGACTGTGAACGTTATAGATGA-3') sur l'activité antitumorale dans un modèle de tumeurs gliales sous-cutanées

### 1. Mode opératoire :

Des cellules de gliome CNS1 cultivées in vitro, sont injectées par voie sous-cutanée à des rats Lewis sains, à raison de 2x10⁶ cellules dans le flanc droit (Kruse C. A. *et al.* (1994), *J. Neurooncol.* **22** pp 191-200).

A J2 après l'injection des cellules tumorales, 50 µg des oligonucléotides possédant les différentes liaisons chimiques sont injectés au niveau du site tumoral et le volume tumoral est mesuré à J10 (groupes traités par un oligonucléotide de liaison : phosphorothioate (PT, n= 9), phosphodiester (PDE, n= 8), méthylphosphonate (MP, n= 9) ; phosphodiester stabilisé en 3' par une base didésoxycytosine (groupe 3', n= 7), ou mixte : phosphodiester avec les trois premières liaisons en 5' et les trois dernières liaisons en 3' du type phosphorothioate (groupe mixte, n= 9). Le groupe témoin reçoit 100 µl de chlorure de sodium (NaCl n= 9).

### 2.Résultats :

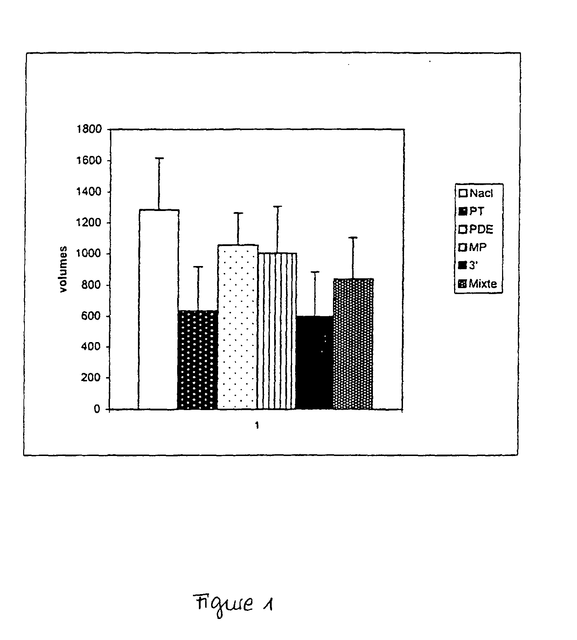

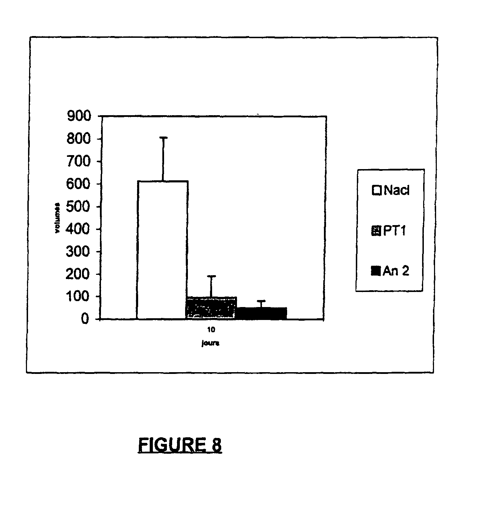

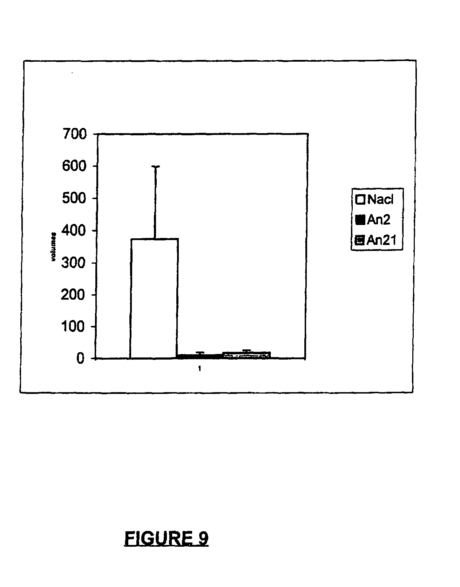

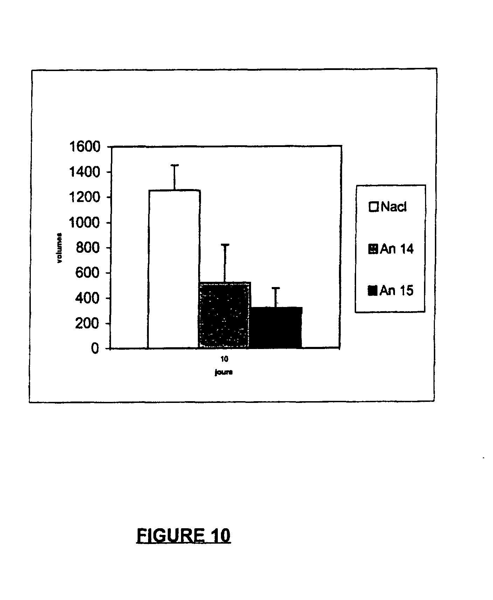

Ils sont illustrés dans la figure 1

Dans ce modèle, les ODN les plus efficaces sont, les oligonucléotides de type phosphorothioate, stabilisés en 3', ou mixte, avec une diminution du volume tumoral de respectivement 50 %, 53 % et 34 %, par rapport au volume des témoins.

### Exemple 13 : Effet des séquences 5'-purine-purine-CG-pyrimidine-pyrimidine -X₁X₂-3' sur la modulation de l'activité antitumorale

### 1. Mode opératoire :

Des cellules de gliome CNS1 cultivées *in vitro,* sont injectées par voie sous-cutanée à des rats Lewis sains, à raison de 2x10⁶ cellules dans le flanc droit (Kruse C. A. *et al.* (1994), *J. Neurooncol.* **22** pp 191-200).

A J2 après l'injection des cellules tumorales, 50 µg des différents oligonucléotides (SEQ ID NO :2 à 13) sont injectés au niveau du site tumoral,et le volume tumoral est mesuré à J10 (figure 8 à 10).

### 2.2.Effet de la séquence du motif hexamérique 5'-purine-purine-CG-pyrimidine-pyrimidine-3' et des séquences adjacentes sur l'efficacité antitumorale des oligonucléotides.

Les résultats sont illustrés à la figure 9

Ils montrent que des oligonucléotides possédant un motif hexamérique différent, GACGTC (An2, SEQ ID NO :8 5'-TGCCAGTGACGTCATGTGAC-3') ou AACGTT (An21, SEQ ID NO :10 5'-TGCCAGTAACGTTATGTGAC-3') et des séquences adjacentes identiques ont la même efficacité antitumorale.

### 2.3. Effet des 2 bases (X₁X₂) adjacentes à la séquence 3' du motif hexamérique 5'-purine-purine-CG-pyrimidine-pyrimidine-3' sur l'efficacité antitumorale

Les résultats sont illustrés à la figure 10.

Ils montrent que les 2 bases adjacentes à la séquence 3' du motif hexamérique, à elles seules, modulent l'efficacité des oligonucléotides puisque 2 oligonucléotides identiques sur toute leur séquence à l'exception de ces 2 nucléotides ont des efficacités différentes, de l'ordre de celles précédemment observées avec les oligonucléotides de l'exemple 2.1. Ainsi, l'oligonucléotide An 14 (SEQ ID NO :3 5'-TGACTGTGAACGTTCCAGATGA-3') est moins efficace que l'oligonucléotide An 15 (SEQ ID NO :9 , 5'-TGACTGTGAACGTTATAGATGA-3'). Les nucléotides AT en 3' du motif hexamérique (An2 (figure 8) et An 15 (figure 10) permettent d'augmenter l'efficacité antitumorale, alors que les nucléotides CC (An 14, figure 10) et CG (PT1, figure 8) ont des effets antitumoraux moins marqués.

## Revendications

1. Utilisation d'oligonucléotides stabilisés qui comprennent au moins un motif octamérique du type AACGTTAT, pour la préparation d'un médicament à action antitumorale.

2. Utilisation selon la revendication 1, **caractérisée en ce que** les oligonucléotides sont choisis dans le groupe constitué par les séquences SEQ ID N° 9, 10, 16, 18, 19, 21, 31, 33, 34, 35 et 47.

3. Utilisation selon l'une quelconque des revendications 1 et 2, **caractérisée en ce qu'**au moins une cytosine du motif octamérique est remplacée par une cytosine modifiée.

4. Utilisation d'oligonucléotides stabilisés selon l'une quelconque des revendications 1 à 3, **caractérisée en ce que** les oligonucléotides stabilisés sont sélectionnés dans le groupe constitué par les phosphorothioates, les phosphorodithioates, les oligonucléotides mixtes phosphodiester-phosphorothioates, les méthylphosphonates et les oligonucléotides dont au moins une extrémité a été stabilisée.

5. Utilisation d'oligonucléotides stabilisés selon l'une quelconque des revendications 1 à 4, **caractérisée en ce que** les oligonucléotides sont sous forme de simple brin ou de double brin.

6. Utilisation d'oligonucléotides stabilisés selon l'une quelconque des revendications 1 à 5, **caractérisée en ce que** les oligonucléotides comprennent au moins 8 nucléotides, et de préférence entre 20 nucléotides et 100 nucléotides.

7. Utilisation d'oligonucléotides stabilisés selon l'une quelconque des revendications 1 à 6, **caractérisée en ce que** les oligonucléotides sont couplés par des liaisons covalentes, ioniques ou faibles à une molécule susceptible d'augmenter l'affinité tumorale.

8. Utilisation d'oligonucléotides stabilisés selon l'une quelconque des revendications 1 à 7, pour la préparation d'un médicament destiné au traitement des cancers chez l'homme, quels que soient leur nature et leur degré d'anaplasie.

9. Utilisation d'oligonucléotides stabilisés selon la revendication 8, **caractérisée en ce que** le cancer est choisi dans le groupe constitué par les cancers des systèmes nerveux central et périphérique.

10. Utilisation d'oligonucléotides stabilisés selon la revendication 8, **caractérisée en ce que** le cancer est choisi dans le groupe constitué par les astrocytomes, les glioblastomes, les médulloblastomes, les neuroblastomes, les mélanomes et les carcinomes.

11. Utilisation d'oligonucléotides stabilisés selon l'une quelconque des revendications 8 à 10, **caractérisée en ce que** le médicament est administré par voie intratumorale.

12. Utilisation d'oligonucléotides stabilisés selon la revendication 11, **caractérisée en ce que** le médicament est administré de manière à avoir une dose de 10 à 1000 µg/g de tumeur, au moins dans une partie de la masse tumorale.

13. Utilisation d' oligonucléotides stabilisés selon l'une quelconque des revendications 8 à 10, **caractérisée en ce que** le médicament est administré par une voie choisie dans le groupe constitué par les voies intraveineuse, intrapéritonéale, topique, transdermique, sous-cutanée, intra-artérielle, pulmonaire, naso-pharyngée ou orale.

14. Utilisation selon l'une quelconque des revendications 8 à 13, **caractérisée en ce que** les oligonucléotides stabilisés sont associés à tout moyen physique ou chimique facilitant l'obtention d'une dose efficace au site tumoral.

15. Utilisation selon l'une quelconque des revendications 8 à 14, **caractérisée en ce que** les oligonucléotides sont administrés sous toute forme pharmaceutiquement acceptable.

16. Utilisation selon l'une quelconque des revendications 8 à 15, **caractérisée en ce que** les oligonucléotides sont associés avec des cellules du système immunitaire, des adjuvants de l'immunité, des cytokines, des anticorps anti-tumoraux, des extraits tumoraux, des antigènes tumoraux ou des cellules tumorales normales, irradiées ou génétiquement modifiées.

17. Utilisation selon l'une quelconque des revendications 1 à 16, **caractérisée en ce que** lesdits oligonucléotides stabilisés comprennent plusieurs motifs octamériques du type AACGTTAT, adjacents ou non.

18. Utilisation selon la revendication 17, **caractérisée en ce que** lesdits oligonucléotides stabilisés comprennent 2 ou 3 motifs octamériques du type AACGTTAT.

## Patentansprüche

1. Verwendung von stabilisierten Oligonukleotiden, die mindestens ein octameres Motiv des Typs AACGTTAT umfassen, zur Herstellung eines Medikaments mit Antitumorwirkung.

2. Verwendung nach Anspruch 1, **dadurch gekennzeichnet, dass** die Oligonukleotide aus der Gruppe bestehend aus den Sequenzen SEQ ID No. 9, 10, 16, 18, 19, 21, 31, 33, 34, 35 und 47 ausgewählt sind.

3. Verwendung nach Anspruch 1 und 2, **dadurch gekennzeichnet, dass** mindestens ein Cytosin des octameren Motivs durch ein modifiziertes Cytosin ersetzt ist.

4. Verwendung von stabilisierten Oligonukleotiden nach einem der Ansprüche 1 bis 3, **dadurch gekennzeichnet, dass** die stabilisierten Oligonukleotide aus der Gruppe bestehend aus Phosphorthioaten, Phosphordithioaten, gemischten Phosphodiester-Phosphorthioat-Oligonukleotiden, Methylphos-phonaten und den Oligonukleotiden, bei denen ein Ende stabilisiert wurde, ausgewählt sind.

5. Verwendung von stabilisierten Oligonukleotiden nach einem der Ansprüche 1 bis 4, **dadurch gekennzeichnet, dass** die Oligonukleotide in Form eines Einzelstrangs oder eines Doppelstrangs vorliegen.

6. Verwendung von stabilisierten Oligonukleotiden nach einem der Ansprüche 1 bis 5, **dadurch gekennzeichnet, dass** die Oligonukleotide mindestens acht Nukleotide und vorzugsweise zwischen 20 Nukleotiden und 100 Nukleotiden umfassen.

7. Verwendung von stabilisierten Oligonukleotiden nach einem der Ansprüche 1 bis 6, **dadurch gekennzeichnet, dass** die Oligonukleotide durch kovalente, ionische oder schwache Bindungen an ein Molekül gekoppelt sind, das geeignet ist, die Tumoraffinität zu erhöhen.

8. Verwendung von stabilisierten Oligonukleotiden nach einem der Ansprüche 1 bis 7 zur Herstellung eines Medikaments, das zur Behandlung von Krebsarten bei Menschen bestimmt ist, die nach ihrer Natur und ihrem Grad zur Anaplasie gehören.

9. Verwendung von stabilisierten Oligonukleotiden nach Anspruch 8, **dadurch gekennzeichnet, dass** der Krebs aus der Gruppe bestehend aus Krebs des zentralen Nervensystems und des peripheren Nervensystems ausgewählt ist.

10. Verwendung von stabilisierten Oligonukleotiden nach Anspruch 8, **dadurch gekennzeichnet, dass** der Krebs aus der Gruppe, bestehend aus Astrocytomen, Glioplastomen, Meduloplastomen, Neuroplastomen, Melanomen und Karzinomen ausgewählt ist.

11. Verwendung von stabilisierten Oligonukleotiden nach einem der Ansprüche 8 bis 10, **dadurch gekennzeichnet, dass** das Medikament auf intratumoralem Weg verabreicht wird.

12. Verwendung von stabilisierten Oligonukleotiden nach Anspruch 11, **dadurch gekennzeichnet, dass** das Medikament derart verabreicht wird, dass eine Dosis von 10 bis 1000 µg/g Gramm Tumor, mindestens in einem Teil der Tumormasse, erhalten wird.

13. Verwendung von stabilisierten Oligonukleotiden nach einem der Ansprüche 8 bis 10, **dadurch gekennzeichnet, dass** das Medikament auf einem Weg verabreicht wird, der aus der Gruppe bestehend aus intravenösem Weg, intraperitonealem Weg, topischem Weg, transdermalem Weg, subkutanem Weg, intraarteriellem Weg, pulmonalem Weg, nasopharyngealem Weg oder oralem Weg ausgewählt wird.

14. Verwendung nach einem der Ansprüche 8 bis 13, **dadurch gekennzeichnet, dass** die stabilisierten Oligonukleotide mit einem physikalischen oder chemischen Mittel assoziiert sind, das den Erhalt einer wirksamen Dosis an der Tumorstelle erleichtert.

15. Verwendung nach einem der Ansprüche 8 bis 14, **dadurch gekennzeichnet, dass** die Oligonukleotide in jeder pharmazeutisch annehmbaren Form verabreicht werden.

16. Verwendung nach einem der Ansprüche 8 bis 15, **dadurch gekennzeichnet, dass** die Oligonukleotide mit Zellen des Immunsystems, Adjuvantien der Immunität, Cytokinen, Antitumorantikörpern, tumoralen Extrakten, tumoralen Antigenen oder normalen Tumorzellen, die bestrahlt oder genetisch modifiziert sind, assoziiert sind.

17. Verwendung nach einem der Ansprüche 1 bis 16, **dadurch gekennzeichnet, dass** die stabilisierten Oligonukleotide mehrere octamere Motive des Typs AACGTTAT, benachbart oder nicht, umfassen.

18. Verwendung nach Anspruch 17, **dadurch gekennzeichnet, dass** die stabilisierten Oligonukleotide zwei oder drei octamere Motive des Typs AACGTTAT umfassen.

## Claims

1. Use of stabilised oligonucleotides comprising at least one octameric unit of the type AACGTTAT, for preparing a medicinal product having antitumoral action.

2. Use according to Claim 1, **characterised in that** the oligonucleotides are selected from the group consisting ofthe sequences SEQ ID Nos. 9, 10, 16, 18, 19, 21, 31, 33, 34, 35 and 47.

3. Use according to either Claim 1 or Claim 2, **characterised in that** at least one cytosine ofthe octameric unit is substituted with a modified cytosine.

4. Use of stabilised oligonucleotides according to any one of Claims 1 to 3, **characterised in that** the stabilised oligonucleotides are selected from the group consisting of the phosphorothioates, the phosphorodithioates, the phosphorodiester-phosphorothioate mixed oligonucleotides, the methylphosphonates and the oligonucleotides at least one end of which has been stabilised.

5. Use of stabilised oligonucleotides according to any one of Claims 1 to 4, **characterised in that** the oligonucleotides are single-stranded or double-stranded.

6. Use of stabilised oligonucleotides according to any one of Claims 1 to 5, **characterised in that** the oligonucleotides comprise at least 8 nucleotides and preferably between 20 nucleotides and 100 nucleotides.

7. Use of stabilised oligonucleotides according to any one of Claims 1 to 6, **characterised in that** the oligonucleotides are linked via covalent, ionic or weak bonds to a molecule capable of increasing the tumoral affinity.

8. Use of stabilised oligonucleotides according to any one of Claims I to 7, for preparing a medicinal product intended for the treatment of human cancers of any kind and any degree of anaplasia.

9. Use of stabilised oligonucleotides according to Claim 8, **characterised in that** the cancer is selected from the group consisting of the cancers of the central and peripheral nervous systems.

10. Use of stabilised oligonucleotides according to Claim 8, **characterised in that** the cancer is selected from the group consisting of the astrocytomas, the glioblastomas, the medulloblastomas, the neuroblastomas, the melanomas and the carcinomas.

11. Use of stabilised oligonucleotides according to any one of Claims 8 to 10, **characterised in that** the medicinal product is administered intratumorally.

12. Use of stabilised oligonucleotides according to Claim 11, **characterised in that** the medicinal product is administered in such a way as to yield a dose of 10 to 1000 µg/g of tumour, at least in a part of the tumour mass.

13. Use of stabilised oligonucleotides according to any one of Claims 8 to 10, **characterised in that** the medicinal product is administered by a route selected from the group consisting of the intravenous, intraperitoneal, topical, transdermal, subcutaneous, intra-arterial, pulmonary, nasopharyngeal and oral routes.

14. Use according to any one of Claims 8 to 13, **characterised in that** the stabilised oligonucleotides are associated with any physical or chemical means of facilitating the achievement of an effective dose at the tumour site.

15. Use according to any one of Claims 8 to 14, **characterised in that** the oligonucleotides are administered in any pharmaceutically acceptable form.

16. Use according to any one of Claims 8 to 15, **characterised in that** the oligonucleotides are associated with cells of the immune system, immunological adjuvants, cytokines, anti-tumoral antibodies, tumoral extracts, tumoral antigens, or normal, irradiated or genetically modified tumour cells.

17. Use according to any one of Claims 1 to 16, **characterised in that** the said stabilised oligonucleotides comprise a plurality of octameric units of the type AACGTTAT, adjacent or otherwise.

18. Use according to Claim 17, **characterised in that** the said stabilised oligonucleotides comprise 2 or 3 octameric units of the type AACGTTAT.
